# EUROPEAN PATENT APPLICATION

(11) **EP 0 823 996 A1**
(43) Date of publication of application: **18.02.1998**
(21) Application number: 97111550.6
(22) Date of filing: 08.07.1997
(51) Int. Cl.: A01N 59/12, A61K 9/00

(54) **Anti-infective against STD relating, for example, to HIV, Chlamydia, gonococcus, treponema pallidum or herpes simplex and device used to preserve/mix principal ingredient and base of such anti-infective**

(30) Priority: 15.08.1996 JP 232632/96; 15.08.1996 JP 232633/96; 14.05.1997 JP 137976/97
(71) Applicant: Yuugen Kaisha Aics Laboratory Sangyo, Beppu-shi, Ohita-ken (JP)
(72) Inventor: Shino, Tsutomu, Beppu-shi, Ohita-ken (JP); Shino, Junko, Beppu-shi, Ohita-ken (JP)
(74) Representative: Zipse + Habersack

(57) **Abstract**

Here are disclosed an anti-infective against STD relating to, for example, to HIV, chlamydia, gonococcus, treponema pallidum or herpes simplex and a novel device comprising a container integrally constructed but adapted to separately preserve/mix a principal ingredient and a base of the anti-infective so as to be easily mixed with each other in actual use of the anti-infective.

The anti-infective contains povidone-iodine as the principal ingredient and high viscosity polymeric solution as the base. The integral container comprises an outer soft container previously filled with the base and an inner container previously filled with the principal ingredient so that the inner container may be unstopped in the actual use of the anti-infective to mix the principal ingredient with the base and the mixture solution may be discharged through a nozzle provided on the outer container.

## Description

### BACKGROUND OF THE INVENTION

### Technical Field

This invention relates to anti-infective consisting of a principal ingredient and a base and adapted to protect mucous membrane of genital organs during sexual act against STD otherwise possibly infected by, for example, HIV, chlamydia, gonococcus, treponema pallidum or herpes simplex and further to a device not only allowing a desired efficacy of the anti-infective to be maintained for a long period by separately preserving the principal ingredient and the base but also allowing said principal ingredient and said base to be mixed together immediately before the anti-infective is actually used.

### Prior Art

It is well known that use of condom is effective as anti-infective measure against HIV. Efficacy of povidone-iodine as the anti-infective against HIV has also been reported in several theses since 1985. Additionally, Japanese Patent Application Disclosure Gazette No. 1989-47717 discloses such anti-infective containing povidone-iodine as the principal ingredient.

Regarding chlamydia, it is well known that tetracycline and macrolide antibiotics are effectively used not only as the anti-infective but also as the therapeutic means.

As for gonorrhea and herpes simplex, specific medicines have recently been developed as the anti-infective against them and it has also become possible to treat them.

Concerning syphilis, development of penicillin has practically exterminated it.

As to the device used to preserve/mix the principal ingredient and the base of said anti-infective against STD, for example, relating to HIV, chlamydia, gonococcus, trepanema pallidum or herpex simplex, the conventional device for such purpose usually comprises a single container within which the principal ingredient and the base have previously been mixed with each other. While it is also well known to preserve the principal ingredient and the base in separate containers so that the principal ingredient and the base may be mixed together immediately before the anti-infective is actually used and thereby a desired effect of the anti-infective may be obtained, none of the devices has been proposed which preserves the principal ingredient and the base in separate but integrally assembled containers allows the principal ingredient and the base to be easily mixed with each other immediately before the anti-infective is actually used.

Once infected with HIV, it is beyond medical treatment and CD₄ lymphocyte is attacked thereby, resulting in immunological deficiency which inevitably deteriorates a biological defense function against various infectious diseases and finally causes a patient's death. Accordingly, prevention of HIV is the best way to be selected for the time being and the most popular method of prevention is the above-mentioned method using condom. However, this popular method is accompanied with several problems. First, regions of genital organs exposed without being sufficiently covered with condom are free from any effective defense. In addition, it is necessary to sterilize the vulva with antiseptic solution when the condom possibly contaminated with HIV is removed after the sexual act. In view of the fact that the condom must be removed directly with user's fingers, it is necessary to sterilize the fingers also and the used condom must be wrapped with oiled paper or plastic before its disposal. The method of prevention using the condom thus imposes the complicated carefulness on the user and there is an apprehension depending on the antiseptic solution used after the sexual act that the mucous membrane of genital organ which is extremely sensitive by nature might suffer from acute pain due to the antiseptic solution.

As the means other than the condom, povidone-iodine is also well known to have an efficacy as the anti-infective against HIV. However, its desired efficacy can not be expected unless a content of povidone-iodine in this anti-infective is at a predetermined level or higher, since its sterilizing effect decreases as the time elapses. While 10% aqueous solution of povidone-iodine inactivates HIV as well as chlamydia as soon as they come in contact with the solution, if the concentration exceeds 10%, the aqueous solution of povidone-iodine often gives the user a stimulant feeling and rarely causes a dangerous side effect of anaphylactic shock as a result of its absorption when the solution is applied to the mucous membrane over a wide region. Furthermore, continuous use of povidone-iodine for a long period adversely affects the thyroid function.

As a countermeasure to avoid the side effect, the residual povidone-iodine on the mucous membrane must be washed with solution of sodium thiosulfate or 1% aqueous solution of ascorbic acid.

While it may be contemplated to combine the condom with the pharmaceutically prepared povidone-iodine, such method is not practical.

As therapeutic methods of STD relating to chlamydia, it is well known to use of antibiotics such as tetracycline antibiotics or macrolide antibiotics. In the male sex, epididymitis occupying approximately one half of non-gonococcal urethritis is reported to be caused by chlamydia. In the female sex, chlamydia is a prophlogistic pathogen for cevicitis, ovaritis and intrapelvic inflammatory disease. There is a danger that the parturient canal infection with chlamydia can cause conjunctivitis or pheumonia in neonatal. In view of the dangerous aspects as have been described above, the prevention of STD during the sexual act is of critical importance.

Use of povidone-iodine is most popular and effective as the principal ingredient of the anti-infective against STD relating, for example, to HIV, chlamydia, gonococcus, treponema pallidum or herpes simplex, as has been mentioned above. While povidone-iodine itself can be effectively preserved for a long period unless it is exposed to radiations such as UV-rays, it must be diluted in water to alleviate its stimulant nature. 1% aqueous solution of povidone-iodine commonly used as the anti-infective is modified and inactivated in a month even at a temperature of 50°C. So far as the aqueous solution of povidone-iodine is supplied along the current route of distribution, the desired efficacy of this solution can not be expected when it attains to the user.

### SUMMARY OF THE INVENTION

In view of the problems as have been described above, it is a principal object of the invention to provide an improved anti-infective against STD relating, for example, to HIV, chlamydia, gonococcus, treponema pallidum or herpes simplex and a novel device to preserve/mix a principal ingredient and a base of such anti-infective comprising a pair of containers separately filled with said principal ingredient and said base, respectively, but integrally assembled so as to facilitate said principal ingredient and said base to be mixed with each other immediately before the anti-infective is actually used.

The object set forth above is achieved, according to an aspect of the invention, by an anti-infective against STD relating, for example, to HIV, chlamydia, gonococcus, treponema pallidum or herpes simplex, characterized by that povidone-iodine in the form of solution or powder is used as a principal ingredient and a high viscosity solution of polymer is used as a base.

Preferably, the principal ingredient comprises 1 ∼ 10% solution of povidone-iodine in water or ethanol. It is also preferable to use the principal ingredient in the form of a mixture of povidone-iodine powder and the other powder of high water-solubility such as sucrose, glucose, lactose, fructose, calcium phosphate, maltose or polyethyleneglycol having a molecular weight of 4000 ∼ 6000. Preferably, the base is prepared by mixing 0.3 ∼ 0.5% of hydroxypropylcellulose and 0.3 ∼ 0.5% of hydroxyethylcellulose with 99.0 ∼ 99.4% of water. Alternatively, the base may be prepared by mixing 0.5% of high viscosity polymer such as high molecular polyethyleneglycol, polysaccharide or sodium polyacrylate each having an average molecular weight of 4000000 ∼ 5000000 with 99.5% of water. The principal ingredient is mixed with the base, immediately before the anti-infective is actually used, at the ratio of 1 : 9.

The inventive device to preserve/mix the principal ingredient and the base of the anti-infective comprises an outer soft container previously filled with the base, and an inner container previously filled with the principal ingredient and placed within said outer soft container so that, immediately before the anti-infective is actually used, said inner container may be stoppered to mix the principal ingredient with the base and then the resultant mixture may be dispensed through a nozzle provided on said outer soft container.

When povidone-iodine in the form of solution is used as the principal ingredient, said inner container is preferably made of light-shielded and colored glass. When povidone-iodine in the form of powder is used as the principal ingredient, on the other hand, said inner container is not limited to said glass container and may be made of synthetic resin.

### BRIEF DESCRIPTION OF THE DRAWINGS

Fig. 1 is a sectional view showing a first embodiment of the inventive preserving/mixing device;
Fig. 2 is a sectional view illustrating a manner in which the first embodiment shown by Fig. 1 operates;
Fig. 3 is a schematic view showing an assembled push lever and stopper;
Fig. 4 is a schematic view showing a glass tube;
Fig. 5 is a sectional view showing the glass tube with the stopper tightly put therein;
Fig. 6 is a sectional view showing a second embodiment of the inventive device;
Fig. 7 is a sectional view illustrating a manner in which the second embodiment shown by Fig. 6 operates;
Fig. 8 is a sectional view showing a third embodiment of the inventive device;
Fig. 9 is a sectional view illustrating a manner in which the third embodiment shown by Fig. 8 operates;
Fig. 10 is a sectional view showing a fourth embodiment of the inventive device;
Fig. 11 is a sectional view illustrating a manner in which the fourth embodiment shown by Fig. 10 operates;
Fig. 12 is a sectional view showing a fifth embodiment of the inventive device;
Fig. 13 is a sectional view illustrating a manner in which the fifth embodiment shown by Fig. 12 operates;
Fig. 14 is a sectional view showing a sixth embodiment of the inventive device;
Fig. 15 is a sectional view illustrating a manner in which the sixth embodiment shown by Fig. 14 operates;
Fig. 16 is a sectional view showing seventh embodiment of the inventive device;
Fig. 17 is a sectional view illustrating a manner in which the seventh embodiment shown by Fig. 16 operates;
Fig. 18 is a sectional view showing an eighth embodiment of the inventive device;
Fig. 19 is a sectional view illustrating a manner in which the eighth embodiment shown by Fig. 18 operates;
Fig. 20 is a sectional view showing a ninth embodiment of the inventive device; and
Fig. 21 is a sectional view illustrating a manner in which the ninth embodiment shown by Fig. 20 operates.

### DESCRIPTION OF THE PREFERRED EMBODIMENT

Since povidone-iodine is soluble in water or ethanol, 1 ∼ 10% of povidone-iodine as solute may be solved in water or ethanol as solvent to obtain the desired principal ingredient. The solution of povidone-iodine used as the principal ingredient is readily affected by radiation such as sun-beam and must be preserved within the light-shielded and colored inner container exclusively provided for the principal ingredient.

The solution of povidone-iodine used for the invention may contain adjuvant such as polyoxyethylenenonylphenyl, concentrated glycerin or lauromacrogol.

It is also possible to use povidone-iodine in the form of powder as the principal ingredient. In this case, the powder of povidone-iodine is mixed with the other powder of high water-solubility, for example, powder of sucrose, glucose, lactose, fructose, calcium phosphate, maltose or polyethyleneglycol (having a molecular weight of 4000 ∼ 6000). Povidone-iodine in the form of powder differs from povidone-iodine in the form of solution in that the former is not inactivated by radiation such as sun-beam and therefore the inner container for the principal ingredient is neither required to be light-shielded and colored nor limited to the container made of glass.

Polymer used as the base is as viscous as being ropy and may be prepared by mixing 0.3 ∼ 0.5% of hydroxypropylcellulose and 0.3 ∼ 0.5% of hydroxyethylcellulose with 99.0 ∼ 99.4% of water. Alternatively, said polymer used as the base may be also obtained by mixing 0.5% of high molecular polyethyleneglycol, polysaccharide or sodium polyacrylate each having an average molecular weight of 4000000 ∼ 5000000 with 99.5% of water. The base is preserved within the outer soft container.

In addition to those which have been described above, there are various types of viscous polymer solution which can be used as the base. The natural water-soluble polymers available for this purpose include xantha-gum, alginic acid and its salt. For example, 0.1 ∼ 0.2% of xantha-gum, alginic acid or its salt may be mixed with 99.8 ∼ 99.9% of water to obtain the desired base.

The semi-synthetic water-soluble polymeric derivatives available for this purpose include hyaluronic acid and cellulose derivative. For example, 0.5 ∼ 1.0% of hyaluronic acid or cellulose derivative may be mixed with 99.0 ∼ 99.5% of water to obtain the desired base.

The synthetic water-soluble polymeric derivatives available for this purpose include polyethyleneglycol, sodium polyacrylate, etc. each having a molecular weight of 4000000 ∼ 5000000. For example, 0.5% or less of polyethyleneglycol having a molecular weight of 4000000 ∼ 5000000 may be mixed with 99% or higher of water to obtain the desired base. Or, 0.1% of sodium polyacrylate may be mixed with 99.9% of water to obtain the desired base.

Like previously described base composed of hydroxypropylcellulose and hydroxyethylcellulose mixed with water, the base classified in this group may be also preserved within the outer soft container.

A volume of the novel anti-infective is preferably 10 ∼ 30ml for every use. Povidone-iodine in the form of solution or powder as the principal ingredient and the viscous solution of polymer as the base are separately preserved at a ratio of 10% : 90% within the inner container and the outer soft container, respectively. These two containers are integrally assembled so that the principal ingredient and the base may be conveniently mixed with each other immediately before the anti-infective is actually used.

### Example 1 (anti-infective)

The anti-infective prepared as has been described hereinabove is poured and applied onto mucous membrane of genital organs such as vulva, vagina and penis immediately before sexual act. It should be understood here that the regions which can not be covered with the condom. It has been found that HIV, chlamydia, gonococcus, treponema pallidum, herpes simplex etc. are perfectly sterilized as soon as several seconds after application. In view of the stability test having indicated that the novel anti-infective with its principal ingredient and base having been mixed together is perfectly modified and inactivated after storage at a temperature of 50°C for one month, the principal ingredient and the base must be separately stored for effective preservation for a long period.

The inventive anti-infective generally contains 1 ∼ 10% of povidone-iodine in the form of solution or powder as has already been described. Now the efficacy of the inventive anti-infective will be considered with respect to the respective types of STD. Gonococcus is sterilized 30 seconds after contact with the novel anti-infective originally containing 1 ∼ 10% of povidone-iodine in the form of solution or powder but diluted in 1/400 for actual application. Virus of herpes simplex is sterilized 15 seconds after contact with the novel anti-infective originally containing 1 ∼ 10% of povidone-iodine in the form of solution or powder but diluted in 1/500 for actual application. HIV can be reliably sterilized within 5 minutes after application of the novel anti-infective containing 10% of povidone-iodine in the form of solution or powder.

A clinical test was conducted for hypersensitivity and allergic response to the novel anti-infective containing 10% of povidone-iodine as the principal ingredient. Specifically, adhesive plaster for patch test "TORII" was impregnated with the novel anti-infective containing 10% of povidone-iodine as the principal ingredient and applied to upper arm of each subject on the inner side for 24 hours and on the outer side for 48 hours. The subjects were classified into a group of 30 healthy persons and a group of 4 patients having anamneses of iodine allergy.

Three patients having anamneses of iodine allergy exhibited reddening reaction which was observed only over the patched regions and completely disappeared 24 hours after the patches had been removed. This suggests that the reddening reaction is not an allergic response but a simple hypersensitive reaction. Accordingly, the novel anti-infective is well compatible even with the patients having anamneses of iodine allergy. Responses of 30 healthy subjects were unexceptionally negative.

In compliance with a request from Mr.TSUTOMU Shino, the inventor of this invention, a series of tests as will be described below were conducted on August, 24, 1995 at Southern Research Institute (under the direction by Mr. Lewis B. Allen).

For these tests, 10g of povidone-iodine was solved in 90ml of ethanol for disinfection according to Japanese Pharmcopoeia to prepare the principal ingredient, on one hand, and 0.4g of hydroxypropylcellulose and 0.4g of hydroxyethylcellulose were solved in 99.2ml of purified water to prepare the base of the anti-infective. Then, 10ml of the principal ingredient was mixed with 90ml of the base.

The anti-infective thus prepared by mixing the principal ingredient with the base was then classified into five groups, i.e., the sample non-diluted with phosphoric buffer, the sample diluted with phosphoric buffer in 1/2, the sample diluted with phosphoric buffer in 1/4, the sample diluted with phosphoric buffer in 1/8 and the sample diluted with phosphoric buffer in 1/100. These respective samples were tested for their effect of inactivating the HIV.

RF staining technique was used to stain HIV-1 and CEM-SS cells were let to be infected with this. The CEM-SS cells thus infected with HIV-1 were added with 50% of phosphoric buffer and 50% of HIV-negative human blood and cultured to observe a HIV-specific morphologic change.

Upon said morphologic change, the respective samples of the anti-infective, i.e., the sample non-diluted with phosphoric buffer, the sample diluted with phosphoric buffer in 1/2, the sample diluted with phosphoric buffer in 1/4, the sample diluted with phosphoric buffer in 1/8 and the sample diluted with phosphoric buffer in 1/100 were poured onto said CEM-SS cells, respectively, and efficacies of these samples were observed over their functioning durations of 5 minutes, 10 minutes and 15 minutes.

As a result, inactivation of HIV-1 was confirmed no matter whether the functioning duration was 5 minutes, 10 minutes or 15 minutes, so far as the samples other than that diluted with phosphoric buffer in 1/100 were concerned. As to the sample diluted with phosphoric buffer in 1/100, no inactivation of HIV-1 was confirmed no matter whether the functioning duration was 5 minutes, 10 minutes or 15 minutes.

### Example 2 (device to preserve/mix the principal ingredient with the base)

Specific embodiments will be given hereunder in reference with the accompanying drawings, now relating to the device according to another aspect of the invention used to preserve/mix the principal ingredient with the base of the above-mentioned anti-infective against STD, for example, due to HIV, chlamydia, gonococcus, treponema pallidum, herpes simplex, etc. It should be understood that the embodiments given hereunder employ povidone-iodine in the form of solution as the principal ingredient and the light-shielded, colored container made of glass as the inner container for the principal ingredient.

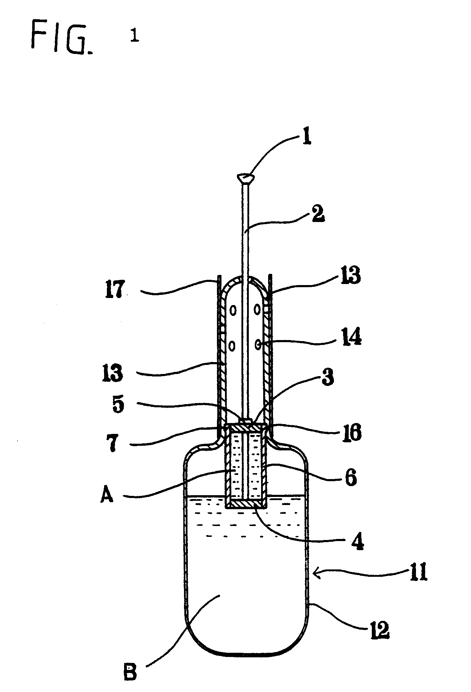

Fig. 1 shows a basic embodiment of the inventive device used to preserve/mix said two components. According to this basic embodiment, the outer soft container 11 in the form of a bottle integrally provided with a nozzle 13 including a plurality of orifices 13. Lower end of this nozzle 13 holds therein a glass container 6 having its upper and lower opened ends normally closed by stoppers 3, 4.

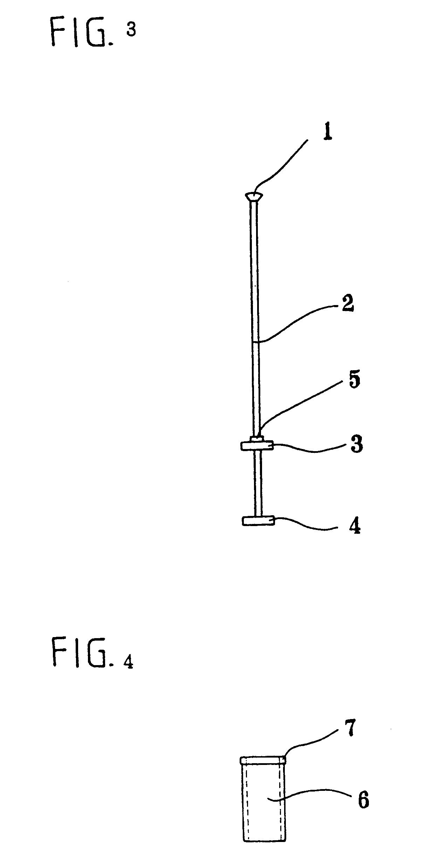

As will be seen in Fig. 4, said glass container 6 is formed around its upper end with a bulge 7. The nozzle 13 as a part of the outer soft container 11 is formed in its lower end with an inner groove 16 and said bulge 7 of the glass container 6 is received by this inner groove 16.

The stoppers 3, 4 used to close the upper and lower openings of the glass container 6 are made of rubber or plastic and, as shown by Fig. 3, fixed around a push lever 2 having a push-button 1 on its top end so that said stoppers 3, 4 may be inserted into the glass container together with the push lever 2 on which said stoppers 3, 4 are fixed until the respective stoppers 3, 4 reliably close the associated openings of the glass container 6. The glass container 6 is filled with the principal ingredient A while said stoppers 3, 4 are inserted into the glass container 6. Stop means 5 fixed on the push lever 2 just above the stopper 3 which will be described later more in detail serves to prevent the stopper 3 from moving relative to the push lever 2 during insertion of the push lever 2 together with this stopper 3.

The bottle-like outer soft container 11 including the nozzle 13 as has been described above has its main portion 12 to be filled with a base B as will be described later. As has already been described, the glass container 6 is filled with the principal ingredient A.

The glass container 6 is light-shielded and colored so that the solution of povidone-iodine which is apt to be affected by radiation may be reliably preserved. The nozzle 13 is covered with film 17.

It should be understood that, if povidone-iodine in the form of powder is employed as the principal ingredient, the inner container is not required to be light-shielded and colored and may be also made of plastic.

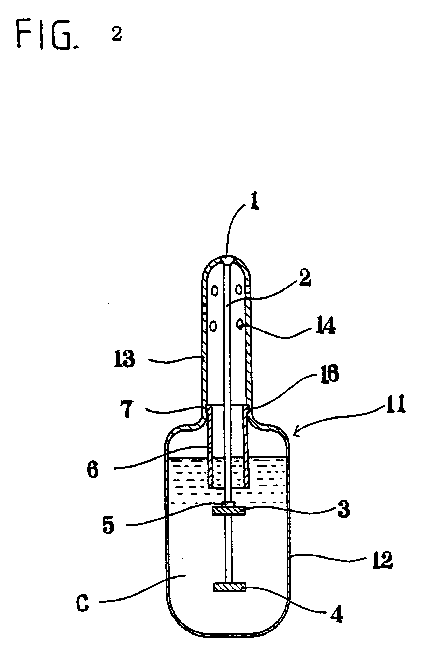

Fig. 2 illustrates a manner in which the embodiment shown by Fig. 1 operates. Operation of the device is started by removing the film 17 entirely covering the nozzle 13. Upon depression of the push-button 1, the stoppers 3, 4 are operatively associated with the push lever 2 to move downward until the principal ingredient A is mixed with the base B within the outer soft container 11. With the nozzle 13 inserted into vagina (not shown), the outer soft container 11 may be squeezed by the hand to introduce mixed solution through the glass container 6 and then through the orifices 14 of the nozzle 13 into vagina so as to achieve washing of vagina as well as disinfection of STD such as HIV.

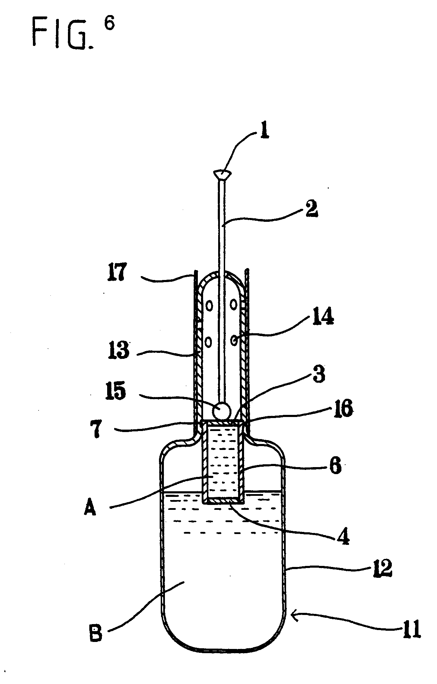

Fig. 6 shows a second embodiment of the invention, in which the stoppers 3, 4 made of rubber or plastic are inserted into the upper and lower ends of the glass container 6 and such assembly is inserted into the outer soft container 11. The bulge 7 formed around the upper end of the glass container 6 is engaged with the groove 16 circumferentially extending between the main portion 12 of the outer soft container 11 and the nozzle 13 having a plurality of orifices 14, on one hand, and the push lever 2 is provided at its lower end with a ball 15, on the other hand, so that the center of the upper stopper 3 may be vertically aligned with the center of said ball 15. The nozzle 13 is entirely covered with the film 17.

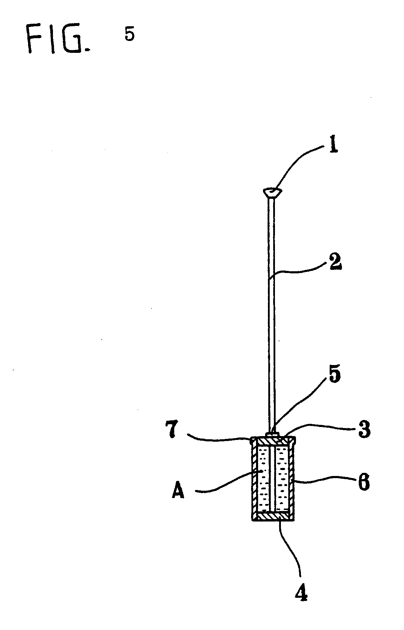

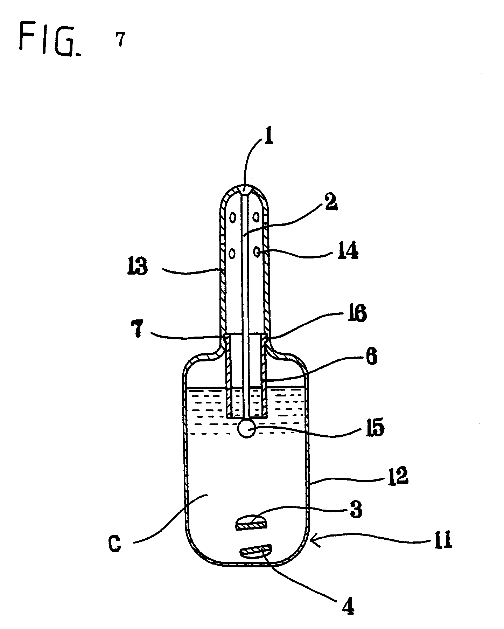

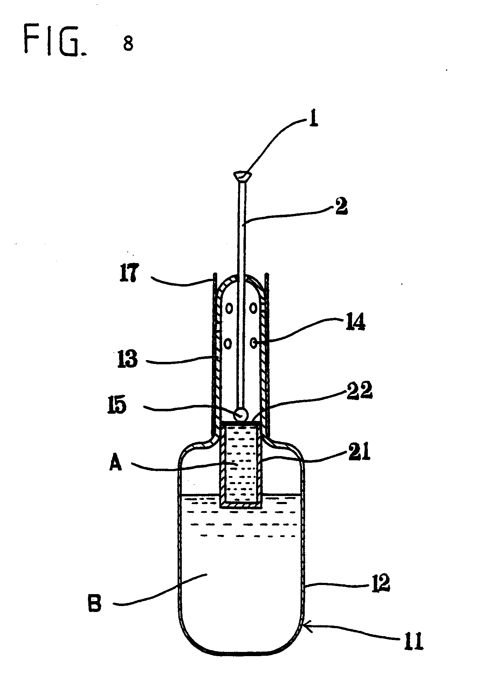

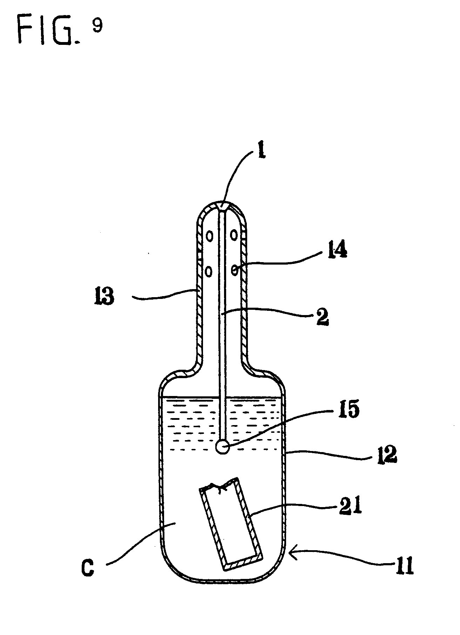

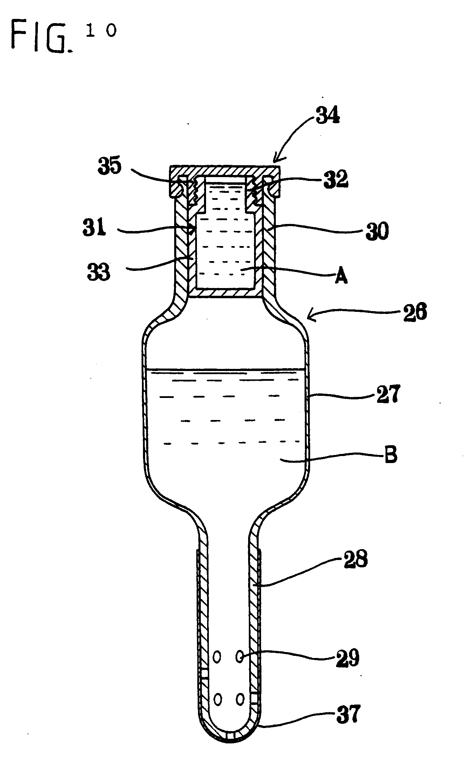

Fig. 7 illustrates a manner in which the second embodiment shown by Fig. 6 operates. Just as in the case shown by Fig. 5, the film 17 is removed and then the push-button 1 is depressed. As the ball 15 depresses the upper stopper 3, a pressure of the principal ingredient A sufficiently increases to thrust the lower stopper 4 out. Consequently, the principal ingredient A is mixed with the base B within the outer soft container 11. The push lever 2 is further depressed until the upper stopper 3 is thrusted out from the glass container 6 and, thereupon, the nozzle 13 may be inserted into vagina, as in the case of Fig. 5, to achieve washing of vagina and disinfection of STD such as HIV.

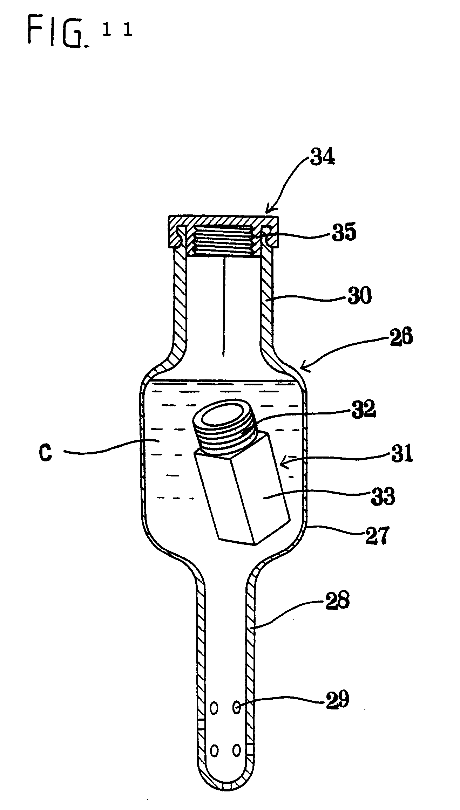

Fig. 8 shows a third embodiment of the invention, in which a cylindrical glass container 21 has its upper end closed by membrane 22 lying between the main portion 12 and the nozzle 13 of the outer soft container 11 and centrally provided with an opening. The push lever 2 is provided at its lower end with the ball 15 so that the center of said ball 15 may be aligned with and rest on said central opening of the membrane 22. The nozzle 13 is entirely covered with the film 17.

Fig. 9 illustrates a manner in which the third embodiment shown by Fig. 8 operates. Upon depression of the push-button 1, the ball forcibly breaks the membrane 22 and then pushes the bottom of the glass container 21 down. With a consequence, the glass containers 21 itself falls and the principal ingredient A is mixed with the base B within the outer soft container 11. Thus, washing of vagina as well as disinfection can be achieved in the same manner as has been described in reference with Fig. 7.

Fig. 10 shows a fourth embodiment of the invention, in which an outer soft container 26 is provided with a main portion 27 adapted to be previously filled with the base B and said main portion 27 is diameter-enlarged with respect to a nozzle 28 and a space 30 of the outer soft container 26. Said space 30 is adapted to hold therein a glass container 31 filled with the principal ingredient A. The space 30 overlies the main portion 27. The glass container 31 is formed around its mouth with an outer screw 32 adapted to be threaded with an inner screw 35 formed around an inner periphery of an outer lid 34 which is snapped around the upper end of the outer soft container 26. It should be understood that a neck of the glass container 31 formed with said outer screw 32 is cylindrical and a main portion 33 of the glass container 31 is in the form of a square pillar. The nozzle 28 underlying the main portion 27 is formed with a plurality of orifices 29 and the nozzle 28 is entirely covered with films 37 so as to prevent the base B from leaking through said orifices 29.

Fig. 11 illustrates a manner in which the fourth embodiment shown by Fig. 10 operates. Rotation of the outer lid 34 causes the outer screw 32 of the glass container 31 is disengaged from the inner screw 35 of the outer lid 34 and the glass container 31 falls into the base filling the outer soft container 26. The principal ingredient A is thus mixed with the base B. Rotation of the outer lid 34 does not cause said outer lid 34 to be detached from the outer soft container 26 and can be detached therefrom only by forcibly pulling it upward. After the principal ingredient A has been mixed with the base B, the film 37 may be removed from the nozzle 28 defining a lower portion of the outer soft container 26, which has been entirely covered with said film 37 and the nozzle 28 may be inserted into vagina as in the case of the previously described embodiments to achieve washing of vagina as well as disinfection.

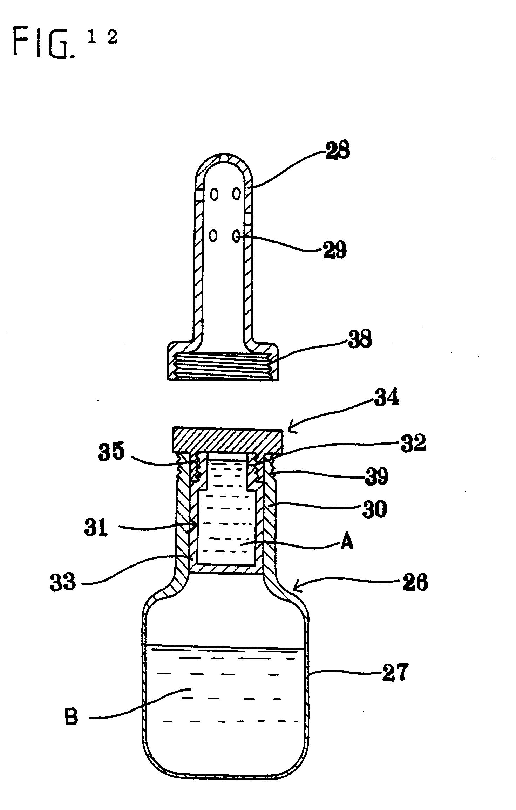

Fig. 12 shows a fifth embodiment of the invention, in which the outer soft container 26 is provided with the main portion 27 adapted to be previously filled with the base B and this main portion 27 is diameter-enlarged with respect to the space 30 overlying said main portion 27 adapted to hold therein the glass container 31 filled with the principal ingredient A. The glass container 31 is formed around its mouth with the outer screw 32 adapted to be engaged with an inner screw 35 circumferentially formed in the outer lid 34 which is snapped around the upper end of the outer soft container 26. As in the case shown by Fig. 10, the neck of the glass container 31 formed with the outer screw 32 is cylindrical and the main portion 33 is in the form of a square pillar. However, this embodiment differs from the fourth embodiment shown by Fig. 10 in that the nozzle 28 provided with a plurality of orifices 29 is not integral with the outer soft container 26 but provided at its lower end with an inner screw 38 adapted to be engaged with an outer screw 39 formed around the upper end of the outer soft container 26.

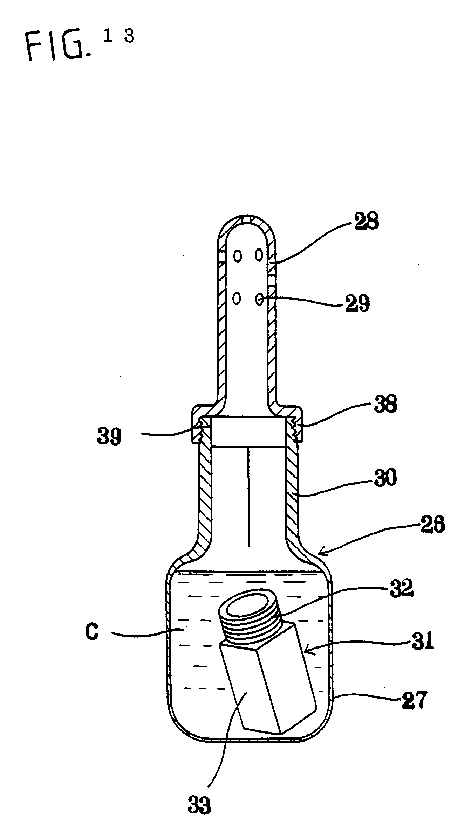

Fig. 13 illustrates a manner in which the fifth embodiment shown by Fig. 12 operates. In the same manner as has been described in reference with Fig. 11, rotation of the outer lid 34 causes the glass container 31 to fall into the base B filling the outer soft container 26 and consequently the principal ingredient A is mixed with the base B. Then, the outer lid 34 is forcibly pulled off and the inner screw 38 formed on the lower end of the nozzle 28 is engaged with the outer screw 39 formed around the upper end of the outer soft container 26. The nozzle 28 thus connected to the outer soft container 26 may be inserted into vagina to achieve washing of vagina as well as disinfection.

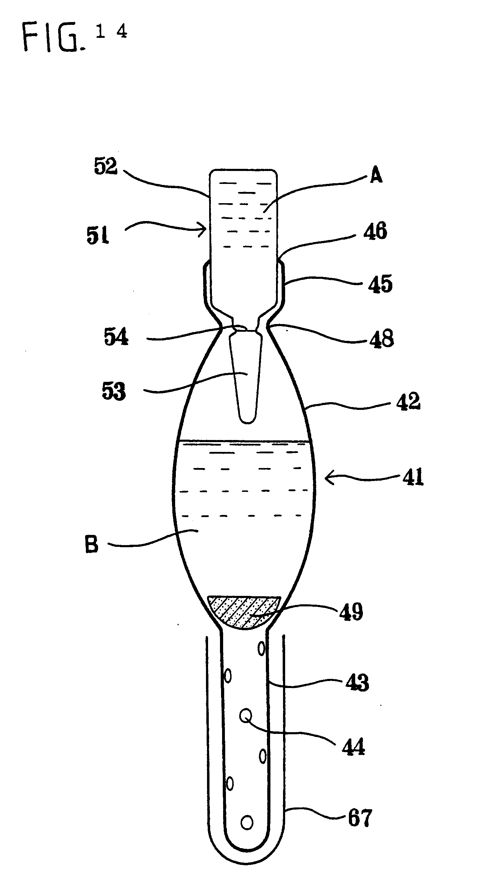

Fig. 14 shows a sixth embodiment of the invention, in which an outer soft container 41 is provided with a main portion 42 adapted to be previously filled with the base B and said main portion 42 is diameter-enlarged with respect to a nozzle 43 as well as an ampule holding space 45 of the outer soft container 41. The ampule holding space 45 overlies the main portion 42 and adapted to hold therein a light-shielded and colored glass ampule 51 filled with the principal ingredient A. The space 45 is provided at its upper end with an opening 46 through which the ampule 51 is inserted into this space 45. After inserted, a head 53 of the ampule 51 lies within the main portion 42 of the outer soft container 41. The ampule 51 has a breakable narrow part 54 between the head 53 and the main portion 52. A nozzle 43 formed with a plurality of orifices 44 forms a lower portion of the outer soft container 41. There is provided filter means 49 between the main portion 42 and the nozzle 43 of the outer soft container 41 and the nozzle 43 is entirely covered with film 67 so as to prevent the base from leaking through said orifices 29.

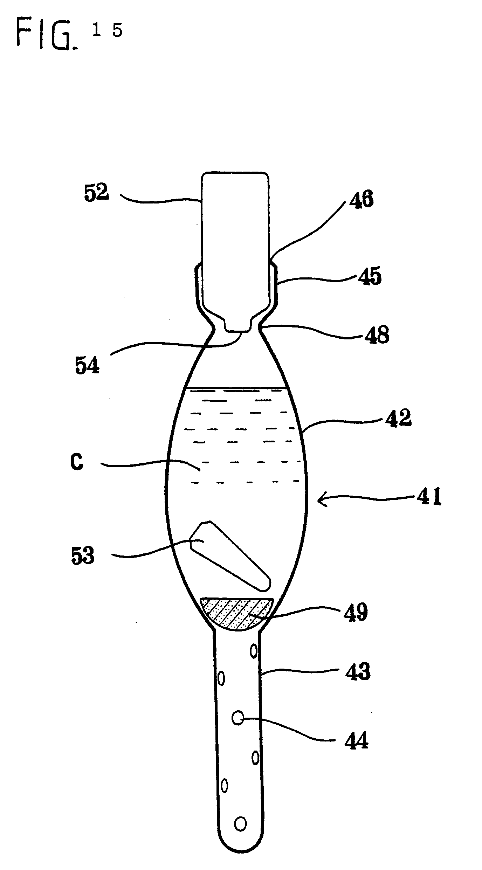

Fig. 15 illustrate's a manner in which the sixth embodiment shown by Fig. 14 operates. The outer soft container 41 is formed with a narrow part 48 between the main portion 42 and the ampule holding space 45 so as to prevent the ampule 51 from falling into the main portion 42. Bending the outer soft container 41 along said narrow part 48 facilitates the ampule 51 to be broken along its narrow part 54 into the head 53 and the main portion 52. Upon separation of the head 53 from the main portion 52, said head 53 falls into the main portion 42 of the outer soft container 41 and the air flowing into the ampule 51 facilitates the principal ingredient A to be poured into the main portion 42 of the outer soft container 41. Consequently, the principal ingredient A is mixed with the base B. Together with the principal ingredient A poured into the main portion 42 of the outer soft container 41, fine pieces of broken glass may fall thereinto. The filter means 49 provided between the main portion 42 and the nozzle 43 assures that only the mixed solution C flows into the nozzle 43 and said fine pieces of broken glass are reliably trapped by the filter means 49. After the mixed solution has been completely transferred into the nozzle 43, the film 67 entirely covering the nozzle 43 may be removed and the nozzle 43 may be inserted into vagina in the same manner as has been described in connection with the previous embodiments to achieve washing of vagina as well as disinfection.

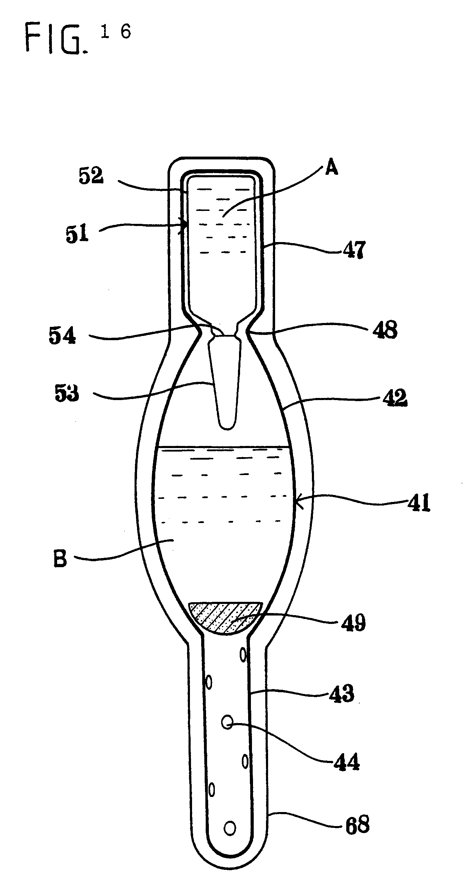

Fig. 16 shows a seventh embodiment of the invention, in which the outer soft container 41 is provided with the main portion 42 adapted to be previously filled with the base B and a space 47 overlying said main portion 42 to hold the ampule 51 previously filled with the principal ingredient A. The outer soft container 41 is entirely covered with film 68 so as to prevent the base B from leaking through a plurality of orifices 44 formed in the nozzle 43. Regarding the remaining aspects, the seventh embodiment is similar to the embodiment shown by Fig. 14.

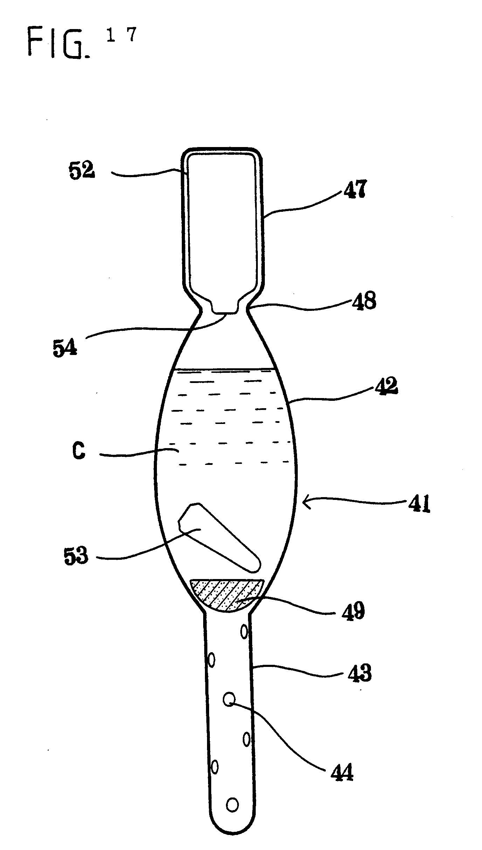

Fig. 17 illustrates a manner in which the seventh embodiment shown by Fig. 16 operates. The manner of operation is similar to that in which the embodiment shown by Fig. 15.

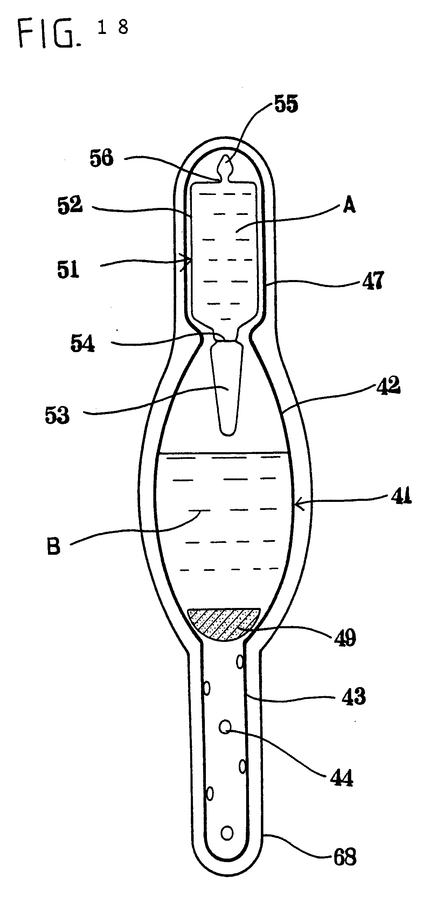

Fig. 18 shows a eighth embodiment of the invention, which is substantially similar to the embodiment shown by Fig. 16 except that the ampule holding space 47 is dimensioned to be larger than that shown by Fig. 16 so that the ampule 51 may be provided on its bottom with a projection 55. This projection 55 is configured so that a narrow part 56 may be formed between the projection 55 and the main portion 52 of the ampule 51.

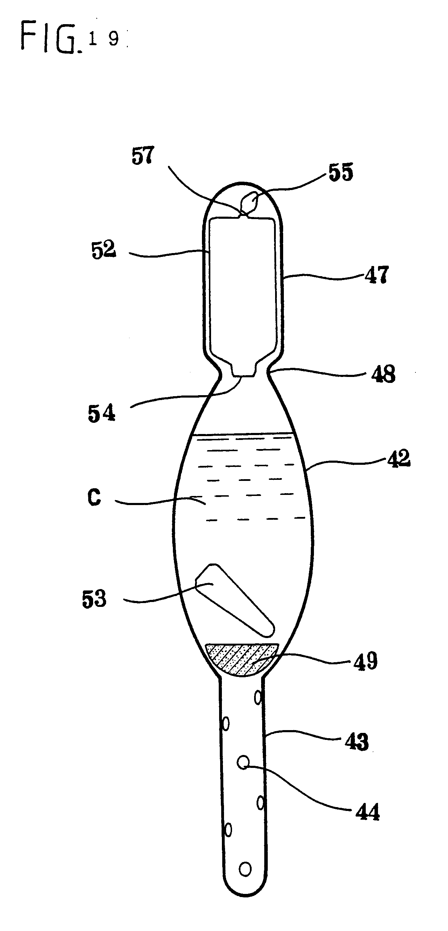

Fig. 19 illustrates a manner in which the eighth embodiment shown by Fig. 18 operates. First, the projection 55 is broken at the narrow part 56 to form a vent hole 57 through which a quantity of air flows into the ampule 51 and then the head 53 is broken at the narrow part 54 to separate the head 53 from the main portion 52. Consequently, the principal ingredient A is facilitated thereby to flow into the main portion 42.

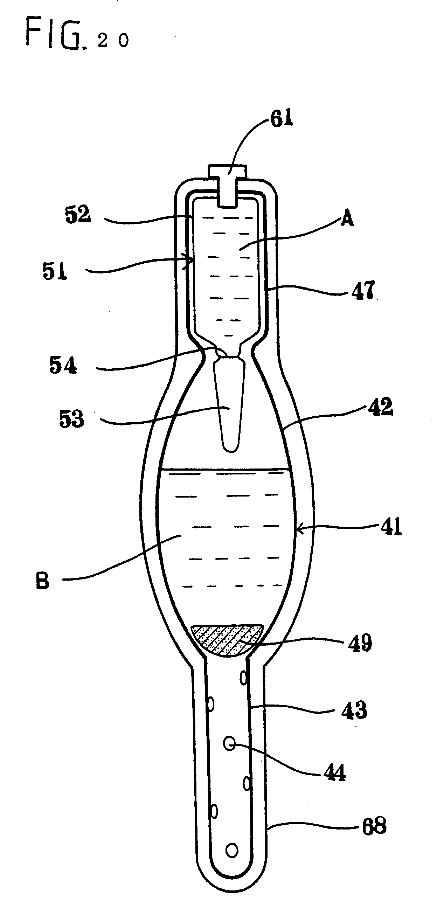

Finally, Fig. 20 shows a ninth embodiment of the invention which is substantially similar to the embodiment shown by Fig. 16 except that the bottom of the ampule 51 and walls of the ampule holding space 47 as well as the film 68 which are closely adjacent said bottom of the ampule 51 are provided with vent aligned holes 57, 50 and 69, respectively, and a common stopper 61 made of plastic or rubber is inserted into these vent holes.

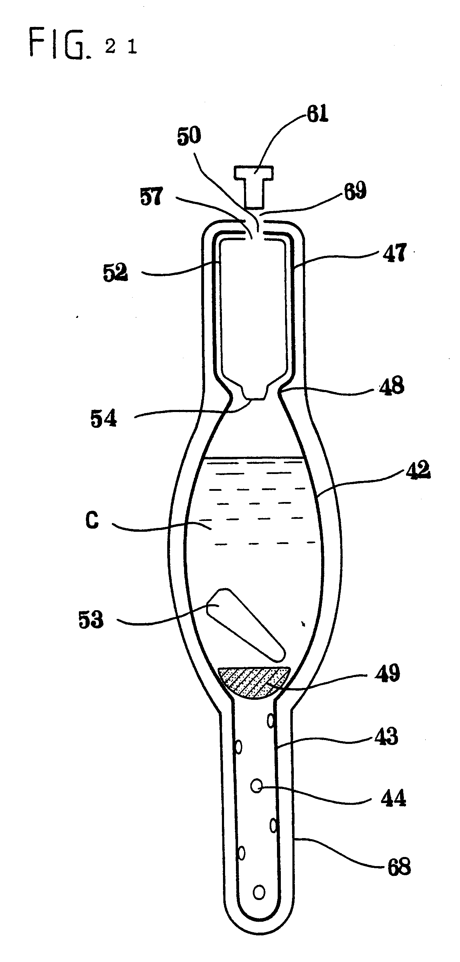

Fig. 21 illustrates a manner in which the ninth embodiment shown by Fig. 20 operates. First, the stopper 61 is removed so as to introduce a quantity of air through the vent holes 57, 50, 69 into the ampule 51. Then, the ampule 51 is broken at the narrow part 54 to separate the head 53 from the main portion 52, whereupon the principal ingredient A is facilitated to flow into the main portion 42.

In actual use, the film 68 may be partially broken and the stopper 61 may be inserted again into the vent holes 57, 50 to achieve washing of vagina gas well as disinfection without an apprehension that the mixed solution C might leak through the orifices 44.

### Effect of the Invention

The novel construction according to the invention provides an effect as will be described below.

As the anti-infective against STD, for example, relating to HIV, chlamydia, gonococcus, treponema pallidum and herpes simplex, povidone-iodine used as the principal ingredient is preferred to ethanol, isopropanol, lysol, formaldehyde and hydrogen peroxide for disinfection in that the povidone-iodine is negligible in its irritative effect to mucous membrane as well as its toxic effect to human body but significant in its disinfective effect. In addition, povidone-iodine is soluble in water as well as in ethanol and available at a low cost. The base is also obtained at a low cost by employing aqueous solution containing hydroxypropylcellulose and hydroxyethylcellulose as solute. Use of such base allows said irritative and toxic effects to be alleviate down to a level exhibited by 1% aqueous solution of povidone-iodine. As will be apparent from said inactivation test, the novel anti-infective practically induces neither hypersensitive nor allergic response and therefore can be used with any apprehension of danger. Furthermore, it is possible for the novel anti-infective to sterilize HIV, chlamydia, gonococcus, treponema pallidum, herpes simplex virus etc. in an extreme short period and to maintain the activity of povidone-iodine for a period enough to disinfect HIV, chlamydia, gonococcus, treponema pallidum, herpes simplex virus etc. Even if mixed with blood, body fluids etc. during actual use of the anti-infective, the desired efficacy of this anti-infective is not affected.

The inventive device is optimal to preserve and mix the anti-infective which can not maintain its efficacy for a long period unless the principal ingredient and the base thereof are separately preserved and exhibit the desired efficacy only when the principal ingredient is mixed with the base immediately before the anti-infective is actually used.

For example, the anti-infective using povidone-iodine as the principal ingredient is inevitably modified and inactivated approximately one month after the principal ingredient was mixed with the base. The device according to the invention comprises the inner container filled with povidone-iodine in the form of 5 ∼ 10% aqueous or ethanol solution or in the form of powder as the principal ingredient and the outer soft container filled with aqueous solution of hydroxypropylcellulose and hydroxyethylcellulose as the base so that, immediately before the anti-infective is actually used, these principal ingredient and the base may be mixed with each other. In this way, the device according to the invention allows the anti-infective to be effectively preserved for a long period and thereby to be effectively used when it is desired to use the anti-infective. Moreover, a plurality of orifices of the nozzle allows the solution of povidone-iodine to be sprayed over the entire wall of vagina. The inventive device can be utilized not only as the device to preserve/mix the principal ingredient and the base of the anti-infective against STD such as due to HIV but also as the device to preserve/mix cosmetics or foods.

## Claims

1. Anti-infective against STD relating, for example, to HIV, chlamydia, gonococcus, treponema pallidum or herpes simplex, characterized by that said anti-infective is high viscosity polymeric solution-based and contains povidone-iodine as a principal ingredient.

2. Anti-infective against STD relating, for example, to HIV, chlamydia, gonococcus, treponema pallidum or herpes simplex according to Claim 1, wherein said anti-infective contains povidone-iodine particularly in the form of solution as the principal ingredient.

3. Anti-infective against STD relating, for example, to HIV, chlamydia, gonococcus, treponema pallidum or herpes simplex according to Claim 1, wherein said anti-infective contains povidone-iodine particularly in the form of powder as the principal ingredient.

4. Anti-infective against STD relating, for example, to HIV, chlamydia, gonococcus, treponema pallidum or herpes simplex according to Claim 2, wherein said povidone-iodine solution contains water as solvent.

5. Anti-infective-against STD relating, for example, to HIV, chlamydia, gonococcus, treponema pallidum or herpes simplex according to Claim 2, wherein said povidone-iodine solution contains ethanol as solvent.

6. Anti-infective against STD relating, for example, to HIV, chlamydia, gonococcus, treponema pallidum or herpes simplex according to Claim 3, wherein said povidone-iodine powder mixed with powder of high water-solubility such as sucrose, glucose, lactose, fructose, calcium phosphate, maltose or polyethyleneglycol having a molecular weight of 4000 ∼ 6000 is used as the principals ingredient.

7. Anti-infective STD relating, for example, to HIV, chlamydia, gonococcus, treponema pallidum or herpes simplex according to any one of claims 1 through 6, wherein said polymeric solution contains natural water-soluble polymer or salt thereof as a solute.

8. Anti-infective against STD relating, for example, to HIV, chlamydia, gonococcus, treponema pallidum or herpes simplex according to any one of Claims 1 through 6, wherein said polymeric solution contains a derivative of semi-synthetic polymer as a solute.

9. Anti-infective against STD relating, for example, to HIV, chlamydia, gonococcus, treponema pallidum or herpes simplex according to any one of Claims 1 through 6, wherein said polymeric solution contains a derivative of synthetic water-soluble polymer.

10. Anti-infective against STD relating, for example, to HIV, chlamydia, gonococcus, treponema pallidum or herpes simplex according to any one of Claims 1 through 9, wherein a ratio-of the principal ingredient to the base is adjusted to 1 : 9.

11. Anti-infective against STD relating, for example, to HIV, chlamydia, gonococcus, treponema pallidum or herpex simplex according to Claim 10, wherein the principal ingredient has a concentration of 5% ∼ 10%.

12. Anti-infective against STD relating, for example, to HIV, chlamydia, gonococcus, treponema pallidum or herpes simplex according to Claim 10, wherein the high viscosity polymeric solution is obtained by mixing 0.3 ∼ 0.5% of hydroxypropylcellulose with 0.3 ∼ 0.5% of hydroxyethylcellulose in 99.0 ∼ 99.4% of water.

13. Anti-infective against STD relating, for example, to HIV, chlamydia, gonococcus, treponema pallidum or herpes simplex according to Claim 10, wherein is obtained by mixing 0.5% of high molecular polyethyleneglycol having an average molecular weight of 4000000 ∼ 5000000, high molecular polysaccharide or sodium polyacrylate in 99.5% of water.

14. Device used to preserve/mix the principal ingredient and the base of the anti-infective against STD relating, for example, to HIV, chlamydia, gonococcus, treponema pallidum or herpes simplex according to any one of Claims 1 through 13, said device comprising an outer soft container previously filled with the base, and an inner container previously filled with the principal ingredient and placed within said outer soft container so that, immediately before use of the anti-infective, said inner container may be unstoppered to mix the principal ingredient with the base and then this mixture may be dispensed through a nozzle provided on said outer soft container.

15. Device used to preserve/mix the principal ingredient and the base of the anti-infective against STD relating, for example, to HIV, chlamydia, gonococcus, treponema pallidum or herpes simplex according to Claim 14, wherein the inner container previously filled with the principal ingredient comprises a cylinder provided at its top and bottom with rubber stoppers which are, in turn, fixed on a push lever, and this assembly of said rubber stoppers and said push lever is inserted into a mouth of the outer soft container so that a push button mounted on a top of said push lever may be pushed down to opened said inner container thereby to mix the principal ingredient with the base and this mixture may be dispensed through the nozzle provided on the outer soft container.

16. Device used to preserve/mix the principal ingredient and the base or the anti-infective against STD relating, for example, to HIV, chlamydia, gonococcus, treponema pallidum or herpes simplex according to Claim 14, wherein the push lever rests on the top of the rubber stopper provided at the top of the cylindrical inner container for the principal ingredient so that said push lever may be depressed and thereby said rubber stopper may be pushed off by a spherical portion formed on the lower end of said push lever to mix the principal ingredient with the base.

17. Device used to preserve/mix the principal ingredient and the base of the anti-infective against STD relating, for example, to HIV, chlamydia, gonococcus, treponema pallidum or herpes simplex according to Claim 14, wherein the push lever rests on a membrane normally covering an opening formed on the top of the inner container so that, upon forcible depression of said push lever, a ball mounted on a lower end of said push lever breaks said membrane and lets said inner container fall to mix the principal ingredient with the base.

18. Device used to preserve/mix the principal ingredient and the base of the anti-infective against STD relating, for example, to HIV, chlamydia, gonococcus, treponema pallidum or herpes simplex according to claim 14, wherein there is provided an outer lid covering a top of the outer soft container, on one hand, and threaded on the inner container, on the other hand, so that said outer lid may be turned to let said inner container fall and thereby to mix the principal ingredient filling said inner container with the base filling said outer soft container before this mixture may be dispensed through the nozzle provided on the outer soft container.

19. Device used to preserve/mix the principal ingredient and the base of the anti-infective against STD relating, for example, to HIV, chlamydia, gonococcus, treponema pallidum or herpes simplex according to Claim 14, wherein an ampule previously filled with the principal ingredient and provided with a breakable line between its head and body is placed within the outer soft container, on one hand, and said outer soft container is correspondingly provided with a narrow part, on the other hand, so that said ampule is broken along said breakable line as said outer soft container is bent at said narrow part and to break said ampule along said breakable line so as to mix the principal ingredient with the base filling said outer soft container; and wherein there is provided filter means within said outer soft container so that only the mixture of said principal ingredient and the base may be dispensed through the nozzle provided on said outer soft container.

20. Device used to preserve/mix the principal ingredient and the base of the anti-infective against STD relating, for example, to HIV, chlamydia, gonococcus, treponema pallidum or herpes simplex according to any one of Claims 14 through 19, wherein said inner container is made of light-shielded and colored glass.
